Europäisches Patentamt

European Patent Office

Office européen des brevets

(19)

(11) Veröffentlichungsnummer: **0 374 289 A1**

## (12) EUROPÄISCHE PATENTANMELDUNG

(21) Anmeldenummer: 88121419.1

(22) Anmeldetag: 21.12.88

(51) Int. Cl.⁵: **H05G 1/52, H01J 35/30, A61B 6/03**

(43) Veröffentlichungstag der Anmeldung:
**27.06.90 Patentblatt 90/26**

(84) Benannte Vertragsstaaten:
**DE FR GB**

(71) Anmelder: **Siemens Aktiengesellschaft
Wittelsbacherplatz 2
D-8000 München 2(DE)**

(72) Erfinder: **Harke, Wolf-Udo, Dr. Dipl.-Phys.
Albert-Schweitzer-Strasse 2
D-6902 Sandhausen(DE)**

(54) **Computertomograph mit periodischer Fokusablenkung.**

(57) Bei einem Computertomographen mit periodischer Ablenkung des Fokus während der Meßwertbildung soll eine störungssichere induktive Ablenkung des Elektronenstrahles der Röntgenröhre erfolgen.

Die Spule (18) für die Fokusablenkung ist in einer Diagonale einer Schaltbrücke angeordnet, die aus vier elektronischen Leistungsschaltern (19 bis 22) aufgebaut ist und mit ihrer anderen Diagonalen an einer Gleichspannungsquelle liegt. Die Leistungsschalter (19 bis 22) sind an vier synchronisierten Ansteuerstufen (23 bis 26) zur wechselweisen Ein- und Ausschaltung jeweils zweier, einander diagonal gegenüberliegender Leistungsschalter (19, 22; 20, 21) angeschlossen.

Die Anwendung erfolgt in einem Computertomographen mit periodisch abgelenktem Fokus.

FIG 3

EP 0 374 289 A1

## Computertomograph mit periodischer Fokusablenkung

Die Erfindung betrifft einen Computertomographen mit einem eine Meßöffnung umschließenden Drehrahmen, auf dem ein von einer Reihe von Detektorelementen gebildeter Strahlendetektor und ein Röntgenstrahler für ein fächerförmiges, auf dem Strahlendetektor auftreffendes Röntgenstrahlenbündel angeordnet sind und welcher zur Durchstrahlung des Untersuchungsobjektes unter verschiedenen Richtungen um eine senkrecht zur Fächerebene liegende, die Meßöffnung durchsetzende Achse drehbar ist, bei dem ein Rechner zur Erzeugung eines Querschnittsbildes des Untersuchungsobjektes aus den mit Hilfe eines Datenerfassungssystems erzeugten Meßwerten des Strahlendetektors vorhanden ist und bei dem eine Ablenkeinheit zur periodischen Ablenkung des Fokus des Röntgenstrahlers in der Fächerebene senkrecht zur Mittelsenkrechten des Strahlendetektors vorgesehen ist, welche eine Spule für die elektromagnetische Ablenkung des Elektronenstrahles von der Kathode zur Anode des Röntgenstrahles aufweist.

In einem Computertomographen der genannten Art werden die Meßwerte durch folgende zwei Parameter beschrieben:

$\alpha$ kennzeichnet die Winkelposition des Fokusschwerpunktes gegenüber einer festen Achse.

$\beta$ ist der Winkel zwischen dem Zentralstrahl des Röntgenstrahlenbündels und der Verbindungslinie zwischen dem jeweiligen Fokus und dem betrachteten Detektorelement.

Wenn der Fokus in dem Drehrahmen eine feste Position hat, also nicht abgelenkt wird, gibt es so viele verschiedene $\beta$-Werte wie Detektorelemente. Bei Realisierung einer periodischen Ablenkung des Fokus um einen Punkt auf der Mittelsenkrechten des Detektors in Richtung senkrecht zu dieser Mittelsenkrechten kann man mit einem bestimmten Detektorelement Meßwerte für mehrere $\beta$-Werte gewinnen, also die Gesamtzahl der Meßwerte und damit die Bildauflösung erhöhen.

Es sei S(t) die mit der Zeit t periodische Auslenkung des Fokus. Dann ist S(t + T) = S(t) und $S(t + \frac{T}{2}) = -S(t)$, wenn T die Periodendauer der Fokusablenkung ist. Der Fokusschwerpunkt im Intervall $0 \leq t \leq \frac{T}{2}$ ist also verschieden von dem Fokusschwerpunkt im Intervall $\frac{T}{2} \leq t \leq T$.

Mit Hilfe von Integratoren kann je Detektorelement ein Meßwert im ersten Intervall und ein weiterer Meßwert im zweiten Intervall gebildet werden. Bei geeigneter Wahl der Amplitude von S(t) in Abhängigkeit von der Form von S läßt sich erreichen, daß die $\beta$-Werte des zweiten Intervalls zwischen den $\beta$-Werten des ersten Intervalls liegen.

Der Erfindung liegt die Aufgabe zugrunde, einen Computertomographen der eingangs genannten Art so auszubilden, daß eine störungssichere induktive Fokusablenkung erfolgt.

Diese Aufgabe ist erfindungsgemäß dadurch gelöst, daß die Spule in einer Diagonale einer Schaltbrücke liegt, die aus vier elektronischen Leistungsschaltern aufgebaut ist, mit ihrer anderen Diagonalen an einer Gleichspannungsquelle liegt und deren Leistungsschalter vier synchronisierte Ansteuerstufen zur wechselweisen Ein- und Ausschaltung jeweils zweier einander diagonal gegenüberliegender Leistungsschalter zugeordnet sind, daß jedem Leistungsschalter je ein Ansteuer-IC zugeordnet ist, daß den Ansteuer-ICs eines Halbbrückenzweiges ein virtuelles Potential zugeordnet ist und daß eine galvanische Trennung der Ansteuer-ICs über Optokoppler erfolgt. Aufgrund der als Wechselrichter wirkenden Schaltbrücke ist ein periodischer Impuls-Wechselstrom in der Spule und damit eine periodische Hin- und Herbewegung des Fokus gewährleistet.

Die Erfindung ist nachfolgend anhand eines in der Zeichnung dargestellten Ausführungsbeispieles näher erläutert. Es zeigen:

Fig. 1 einen Computertomographen nach der Erfindung,

Fig. 2 eine Einzelheit des Computertomographen nach Fig. 1, und

Fig. 3 eine Schaltungseinzelheit des Computertomographen nach den Fig. 1 und 2.

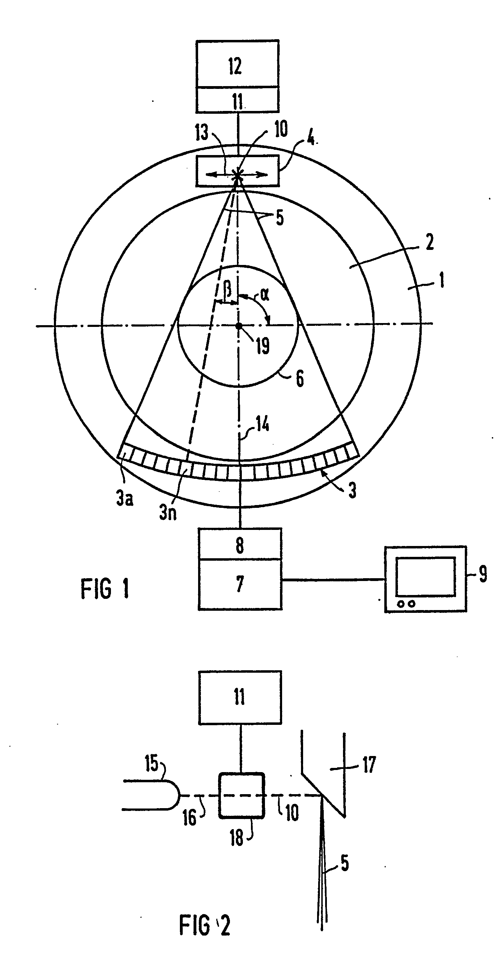

In der Fig. 1 ist ein Drehrahmen 1 dargestellt, der eine Meßöffnung 2 umschließt und einen aus einer Reihe von Detektorelementen 3a usw. bestehenden Strahlendetektor 3 sowie einen Röntgenstrahler 4 für ein fächerförmiges, auf dem Strahlendetektor 3 auftreffendes Röntgenstrahlenbündel 5 trägt. Der Drehrahmen 1 ist zur Durchstrahlung eines Meßfeldes 6, in dem sich ein Untersuchungsobjekt, z.B. ein Patient auf einer Liege, befindet, unter verschiedenen Richtungen um eine Achse 19 drehbar, welche senkrecht zur Fächerebene des Röntgenstrahlenbündels 5 die Meßöffnung 2 und damit das Meßfeld 6 durchsetzt. Ein Rechner 7 erzeugt aus den dabei mit Hilfe eines Datenerfassungssystems 8 gewonnenen Meßwerten des Strahlendetektors 3 ein Querschnittsbild des Untersuchungsobjektes. Dieses wird auf einem Sichtgerät 9 wiedergegeben.

Der Fokus 10 des Röntgenstrahlers 4 wird zur Erhöhung der Zahl der Meßwerte mit Hilfe einer Ablenkeinheit 11 in einem Röntgengenerator 12 periodisch in Richtung des Doppelpfeils 13, also senkrecht zur Mittelsenkrechten 14 des Strahlendetektors 3, abgelenkt.

In Fig. 1 ist für einen Fokusschwerpunkt der Winkel $\alpha$ und für das Detektorelement 3n der Winkel β eingezeichnet.

Die Fig. 2 zeigt den Aufbau des Röntgenstrahlers 4 zur periodischen Ablenkung des Röntgenstrahlenbündels 5. Eine Kathode 15 sendet einen Elektronenstrahl 16 auf eine Anode 17, von der das Röntgenstrahlenbündel 5 so ausgeht, daß die Fächerebene senkrecht zur Zeichenebene liegt. Mit Hilfe einer Ablenkspule 18, die an der Ablenkeinheit 11 angeschlossen ist, erfolgt die periodische Fokusablenkung senkrecht zur Zeichenebene.

Zur kontinuierlichen periodischen Ablenkung des Röntgenstrahlenbündels 5 des als Drehanoden-Röntgenröhre ausgebildeten Röntgenstrahlers 4 wird der die Röntgenquanten erzeugende Elektronenstrahl durch ein variables Magnetfeld abgelenkt.

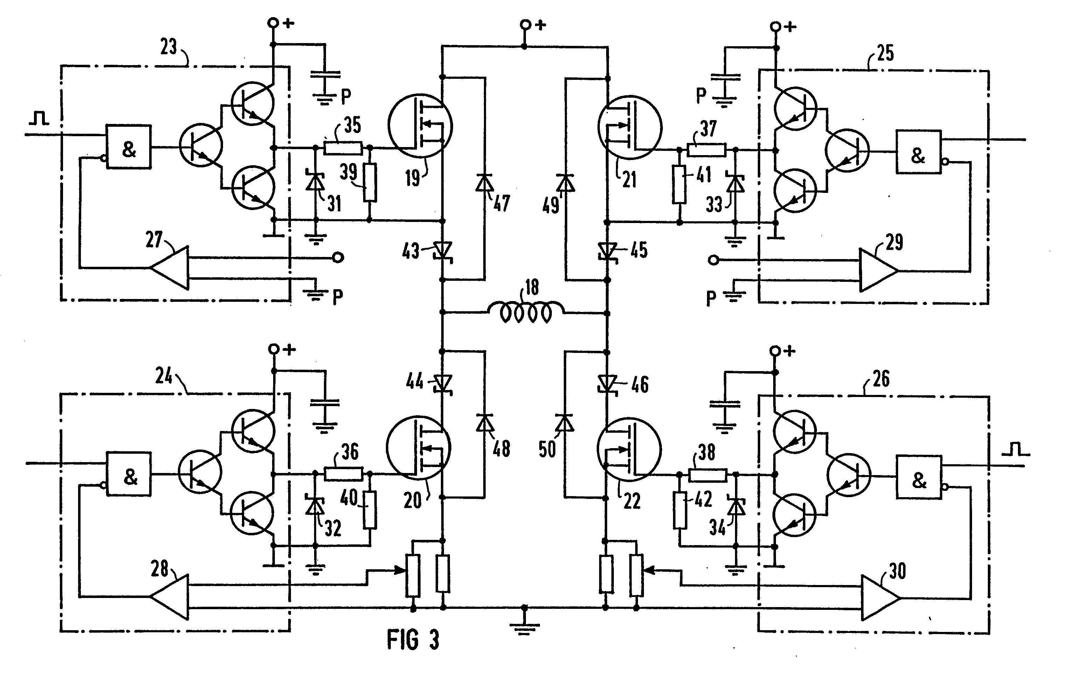

Die Spule 18 ist gemäß Fig. 3 als Last in einer Brücke eingebaut und wird von Strom wechselnder Polarität durchflossen. Die Brücke besteht aus elektronischen Leistungsschaltern 19 bis 22 (MOSFETs). Über die Versorgungsspannung (70 V) wird der das Magnetfeld erzeugende Strom hervorgerufen und fließt bei wechselnder Ansteuerung der jeweils diagonal gegenüberliegenden Leistungsschalter 19, 22 bzw. 20, 21 durch den jeweiligen Brückenzweig.

Die Schaltung besitzt eine nur wenige Nanosekunden betragende Totzeit. Ströme bis zu 60 A lassen sich mit einer Totzeit von ca. 2 ns und damit hohem elektronischem Tastverhältnis schalten.

Ansteuer-ICs 23 bis 26 bewirken, daß Überströme durch jeweils einen monolithisch eingebauten Komparator 27 bis 30 schnell unterbunden werden. Dadurch wird eine hohe Zuverlässigkeit der Schaltung gewährleistet.

Die synchronisierten Ansteuer-ICs 23 bis 26 sind so ausgebildet, daß in weniger als 60 ns große Ansteuerströme von 1,5 A bis 3 A zur Verfügung stehen, um den jeweiligen Brückenzweig mit Strom zu versorgen. Dabei ist es sinnvoll, jeweils eine ultraschnelle Schottky-Diode 31 bis 34 mit ihrer Anode nach Masse vor den Gate-Elektroden der Leistungsschalter 19 bis 22 einzubauen.

Die Schottky-Dioden 31 bis 34 verhindern, daß ein negatives Unterschwingen der Ausgangsspannung der Ansteuer-ICs 23 bis 26 unter Masse erfolgen kann. Wesentlich kommt es hier darauf an, daß jede Schottky-Diode 31 bis 34 eine maximale Schwellwertspannung von 0,3 V bei einem Strom von ca. 0,2 A aufweist. Dann ist ein wirkungsvoller Strompfad bei Unterschwingen der Ansteuerspannung gewährleistet. In der Praxis sollten die ultraschnellen Schottky-Dioden 31 bis 34 so dicht wie möglich an den Ansteuer-ICs 23 bis 26 eingebaut werden.

Zusätzlich können Widerstände 35 bis 38 von 10 Ohm ... 20 Ohm vor den Gate-Elektroden der Leistungsschalter 19 bis 22 eingebaut werden, um den Ansteuerstrom zu begrenzen. Positives Überschwingen, hervorgerufen durch unerwünschte Oszillationen, läßt sich auf diese Weise wirkungsvoll dämpfen. Dabei ist jedoch darauf zu achten, daß sich unter Umständen durch zu hohe Werte dieser Widerstände 35 bis 38 die schnellen Ansteuerflanken der Ansteuer-ICs verlangsamen.

Weiterhin muß beim Aufbau der Schaltung dafür gesorgt werden, daß nur sehr kleine parasitäre Induktivitäten in der Nähe der Ansteuer-ICs 23 bis 26 entstehen.

Bei der Auslegung der Vollbrückenschaltung sollen kurze Totzeiten, hohe Ströme und hohe Taktfrequenzen erreicht werden. Um diese Ziele realisieren zu können, müssen Leistungsschalter 19 bis 22 mit einer großen Vorwärtssteilheit verwendet werden, die ihrerseits von Ansteuer-ICs 23 bis 26 mit hoher Anstiegssteilheit angesteuert werden. Ohne die Widerstände 35 bis 38 wird der von den Ansteuer-ICs 23 bis 26 erzeugte Spitzenstrom nur durch die Gate-Kapazität der Leistungsschalter 19 bis 22 und das Verhältnis dU/dt ihres Totem-Pole-Ausganges begrenzt.

Die Ansteuer-ICs 23 bis 26 sind derart aufgebaut, daß in ihnen Kreuzleitungseffekte kaum auftreten können. Auch ist der ausgeschaltete Zustand an ihren Ausgängen gewährleistet. Durch Widerstände 39 bis 41 wird bei Nichtanliegen der Ansteuerspannung das betreffende Gate der Leistungsschalter 19 bis 22 definiert auf Masse gezogen.

Hierbei ist zu beachten, daß die Ansteuerspannung für die Ansteuer-ICs 23 bis 26 zumindest im oberen Teil der Vollbrücken schaltung über Trenntransformatoren oder Optokoppler zugeführt wird. Es erfolgt in jedem Fall eine variable Potentialverschiebung des gegebenenfalls virtuellen Massebezugspunktes des oberen Schaltungsteiles. Dabei ist es erforderlich, daß die Versorgungsspannung für die Ansteuer-ICs 23 bis 26 stets über Massepotential liegt (+ 5 V ... + 40 V). Die Gate-Kapazität parallelgeschalteter Leistungsschalter ist zum Teil wesentlich höher als die einzelner Leistungsschalter. Trotzdem sind die Stromanstiegsflanken an den Gate-Elektroden steil genug und die Ströme ausreichend groß, um die Leistungsschalter schnell durchzuschalten. Typische Durchschaltzeiten liegen bei ca. 60 ns.

Es sollen noch einige Gründe genannt werden, die für eine Parallelschaltung von einzelnen Leistungsschaltern anstelle der Leistungsschalter 19 bis 22 sprechen. Dabei bewirkt der verringerte Durchlaßwiderstand (Onwiderstand) einen geringeren Leistungsverlust in getakteten Schaltungen. Zusätzlich wird durch die Parallelschaltung die Schaltinduktivität herabgesetzt. Darüber hinaus weist die gesamte Schaltung ein verbessertes thermisches Verhalten auf. Auch kann der Drainstrom höhere Werte annehmen. Sollten Strahlungseffekte kompensiert werden, so ist ebenfalls eine Parallelschaltung einzelner Leistungsschalter für jeden Leistungsschalter 19 bis 22 sinnvoll.

Im Idealfall müßten alle parallelgeschalteten Leistungsschalter in ihren elektrischen Parametern identisch sein. Jedoch treten fertigungsbedingte Streuungen bezüglich des Onwiderstandes, der Vorwärtssteilhaft gfs, der Schwellwertspannung Ut und der Eingangskapazität Cg auf.

Am stärksten macht sich jedoch eine Streuung bezüglich Ut bemerkbar. Durch unterschiedlich hohe Schwellwertspannungen Ut fließen in parallelgeschalteten Leistungsschaltern unterschiedlich große Ströme. Wird dieser Unterschied zu groß, entsteht sehr schnell eine thermische Überlastung des betreffenden Leistungsschalters mit dem kleinsten Ut. Diese Überlastung kann zu einer Zerstörung des betreffenden Leistungsschalters führen.

Jedoch kann dieser unerwünschte Effekt hinreichend gut reduziert werden, indem man die Schaltgeschwindigkeit des Ansteuer-ICs 23 bis 26 entsprechend erhöht. Dadurch wird die Zeit verkürzt, in der die Gate-Kapazität geladen und entladen wird. In jedem Fall ist es sinnvoll, durch geeignete Wahl eines Ansteuer-ICs mit genügend steiler Ansteuerflanke das dU/dt zu erhöhen.

Es sollte an dieser Stelle darauf hingewiesen werden, daß sich auch in Vollbrückenschaltungen die auf einem Leistungsschalter 19 bis 22 befindliche parasitäre Diode (Body-Diode) negativ auswirkt. Die Sperrerholzeit dieser Diode ist im allgemeinen wesentlich länger als die Schaltzeit des eigentlichen Leistungsschalters. Jedoch wird die Drain-Source-Durchbruchspannung durch diese Body-Diode definiert. Deshalb muß vor allem in einer Vollbrückenschaltung mit induktiver Last (Spule 18) an eine Schutzbeschaltung der Leistungsschalter 19 bis 22 gedacht werden.

Diese Schutzbeschaltung kann wirkungsvoll durch den Einbau einer sehr schnellen Schottky-Diode 43 bis 46 und einer ultraschnellen Freilaufdiode 47 bis 50 mit extrem kurzer Sperrerholzeit erfolgen. Dabei befinden sich die Schottky-Dioden 43 bis 46 in der Schaltung vor den Source-Elektroden der parallelgeschalteten Leistungsschalter. Die ultraschnelle Freilaufdiode 47 bis 50 ist den parasitären Dioden unmittelbar parallelgeschaltet. Induktive Spitzen, die durch das Schalten der Spule 18 entstehen, können auf diese Weise weitgehend von den Leistungsschaltern 19 bis 22 ferngehalten werden. Der Sinn und Zweck der Schottky-Dioden 43 bis 46 besteht darin, daß verhindert wird, den induzierten Strom durch die parasitären Dioden fließen zu lassen. Die ultraschnellen Freilaufdioden 47 bis 50 stellen einen wirkungsvollen Strompfad für diesen rückwärts fließenden Strom dar.

Wesentlich für die beschriebene Schaltung ist, daß den Ansteuer-ICs 23, 25 im oberen Halbbrückenzweig ein virtuelles Potential P als Bezugspotential zugeordnet ist. Die Versorgungsspannung beträgt + 70V und übersteigt die normalerweise höchstzulässige Betriebsspannung von maximal + 40V der Ansteuer-ICs. Eine erforderliche galvanische Trennung der Ansteuer-ICs 23 bis 26 kann über konventionelle Optokoppler erfolgen und verhindert eine Zerstörung der Ansteuer-ICs 23 bis 26.

## Ansprüche

1. Computertomograph mit einem eine Meßöffnung (2) umschließenden Drehrahmen (1), auf dem ein von einer Reihe von Detektorelementen (3a ...3n ...) gebildeter Strahlendetektor (3) und ein Röntgenstrahler (4) für ein fächerförmiges, auf dem Strahlendetektor (3) auftreffendes Röntgenstrahlenbündel (5) angeordnet sind und welcher zur Durchstrahlung des Untersuchungsobjektes unter verschiedenen Richtungen um eine senkrecht zur Fächerebene liegende, die Meßöffnung (2) durchsetzende Achse (19) drehbar ist, bei dem ein Rechner (7) zur Erzeugung eines Querschnittsbildes des Untersuchungsobjektes aus den mit Hilfe eines Datenerfassungssystems (8) erzeugten Meßwerten des Strahlendetektors (3) vorhanden ist und bei dem eine Ablenkeinheit (11) zur periodischen Ablenkung des Fokus (10) des Röntgenstrahlers (4) in der Fächerebene senkrecht zur Mittelsenkrechten (14) des Strahlendetektors (3) vorgesehen sind, welche eine Spule (18) für die elektromagnetische Ablenkung des Elektronenstrahles (16) von der Kathode (15) zur Anode (17) des Röntgenstrahlers (4) aufweist, **dadurch gekennzeichnet, daß** die Spule (18) in einer Diagonale einer Schaltbrücke liegt, die aus vier elektronischen Leistungsschaltern (19 bis 22) aufgebaut ist, mit ihrer anderen Diagonalen an einer Gleichspannungsquelle liegt und deren Leistungsschalter (19 bis 22) vier synchronisierte Ansteuerstufen (23 bis 26) zur wechselweisen Ein- und Ausschaltung jeweils zweier einander diagonal gegenüberliegender Leistungsschalter (19, 22; 20, 21) zugeordnet sind, daß jedem Leistungsschalter (19 bis 22) je ein Ansteuer-IC (23 bis 26) zugeordnet ist, daß den Ansteuer-ICs (23, 25) eines Halbbrückenzweiges ein virtuelles Potential (P) zugeordnet ist und daß eine galvanische Trennung der Ansteuer-ICs (23 bis 26) über Optokoppler erfolgt.

2. Computertomograph nach Anspruch 1, **dadurch gekennzeichnet, daß** am Ausgang jedes Ansteuer-ICs (23 bis 26) je eine Schottky-Diode (31 bis 34) liegt.

3. Computertomograph nach Anspruch 1, **dadurch gekennzeichnet, daß** jedem Leistungsschalter (19 bis 22) je ein Ansteuer-IC (23 bis 26) zugeordnet ist und daß am Ausgang jedes Ansteuer-ICs (23 bis 26) je eine Schottky-Diode (31 bis 34) liegt.

4. Computertomograph nach Anspruch 2, **dadurch gekennzeichnet,** daß zwischen den Leistungsschaltern (19 bis 22) und den Ansteuer-ICs (23 bis 26) Koppelwiderstände (35 bis 38) von 10 Ohm bis 20 Ohm liegen.

5. Computertomograph nach einem der Ansprüche 1 bis 3, **dadurch gekennzeichnet,** daß zwischen dem Gate und Masse jedes Leistungsschalters (19 bis 22) je ein Widerstand (39 bis 42) von etwa 10 kOhm liegt.

6. Computertomograph nach einem der Ansprüche 1 bis 4, **dadurch gekennzeichnet,** daß jeder Leistungsschalter (19 bis 22) aus der Parallelschaltung mehrerer einzelner Leistungsschalter besteht.

7. Computertomograph nach einem der Ansprüche 1 bis 5, **dadurch gekennzeichnet,** daß jeder Leistungsschalter (19 bis 22) in Reihe mit je einer schnellen Schottky-Diode (43 bis 46) liegt und daß parallel zu jeder dieser Reihenschaltungen (19, 43; 20, 44; 21, 45; 22, 46) je eine ultraschnelle Freilaufdiode (47 bis 50) geschaltet ist.

FIG 1

FIG 2

FIG 3

EP 0 374 289 A1

88 P 3553 E

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 323 779 (R.D. ALBERT)<br>* Spalte 4, Zeilen 3-17; Spalte 23, Zeilen 17-63; Spalte 26, Zeilen 11-50; Figuren 13-15 *<br>--- | 1 | H 05 G 1/52<br>H 01 J 35/30<br>A 61 B 6/03 |
| A | US-A-4 045 672 (E. WATANABE)<br>* Spalte 5, Zeilen 19-57; Figur 10 *<br>--- | 1 | |
| A | US-A-4 688 241 (R.S. PEUGEOT)<br>* Spalte 4, Zeilen 15-55; Spalte 9, Zeilen 19-65; Figuren 2,8,9 *<br>--- | 1 | |
| A | US-A-4 057 745 (R.D. ALBERT)<br>* Spalte 10, Zeilen 2-35; Spalte 25, Zeilen 14-61; Figur 14 *<br>--- | 1 | |
| A | US-A-4 392 235 (Z.M. HOUSTON)<br>* Spalte 3, Zeilen 20-65; Spalte 5, Zeile 59 - Spalte 6, Zeile 40; Figuren 1,4,5 *<br>----- | 1 | |

**RECHERCHIERTE SACHGEBIETE (Int. Cl.5)**

A 61 B
G 01 N
H 01 J
H 05 G

Der vorliegende Recherchenbericht wurde für alle Patentansprüche erstellt

| Recherchenort | Abschlußdatum der Recherche | Prüfer |
|---|---|---|
| DEN HAAG | 10-08-1989 | HORAK G.I. |

EPO FORM 1503 03.82 (P0403)